# EUROPÄISCHE PATENTSCHRIFT

(11) **EP 2 822 673 B1**
(45) Veröffentlichungstag und Bekanntmachung des Hinweises auf die Patenterteilung: **10.08.2016**
(21) Anmeldenummer: 13718778.7
(22) Anmeldetag: 08.03.2013
(51) Int. Cl.: B01D 53/14

(54) **VERFAHREN UND ANLAGE ZUR ABTRENNUNG VON KOHLENDIOXID AUS BIOGAS**
METHOD AND INSTALLATION FOR SEPARATING CARBON DIOXIDE FROM BIOGAS
PROCÉDÉ ET INSTALLATION PERMETTANT DE SÉPARER DU DIOXYDE DE CARBONE DE BIOGAZ

(30) Priorität: 09.03.2012 DE 102012101991
(43) Veröffentlichungstag der Anmeldung: 14.01.2015
(73) Patentinhaber: DGE Dr.-Ing. Günther Engineering GmbH, 06886 Wittenberg (DE)
(72) Erfinder: GÜNTHER, Lothar, 82538 Geretsried (DE)
(74) Vertreter: Weidner Stern Jeschke
(86) Internationale Anmeldenummer: PCT/DE2013/100087
(87) Internationale Veröffentlichungsnummer: WO 2013/131517

(56) Entgegenhaltungen:
- WO-A1-97/03920
- WO-A1-2010/019763
- US-A- 5 820 837
- US-A- 5 842 357
- US-A1- 2010 319 540

## Beschreibung

Die Erfindung bezieht sich auf ein Verfahren zur Abtrennung von Kohlendioxid aus Biogas mittels Druckwäsche unter Verwendung eines physikalisch wirkenden Lösungsmittels, wobei die verunreinigte Waschlösung nach erfolgter Regeneration im Kreislauf gefahren wird. Ferner bezieht sich die Erfindung auf eine geeignete Anlage zur Durchführung des Verfahrens.

Unter Biogas sind alle Gase zu verstehen, die durch anaerobe Vergärung, insbesondere von biologisch abbaubaren landwirtschaftlichen Produkten, Rückständen und Abfällen pflanzlichen und/oder tierischen Ursprungs, entstehen, also auch Klar- und Deponiegase. Druckwäscheverfahren zur Entfernung von Kohlendioxid aus Biogas beruhen auf dem Wirkprinzip der Lösung von Gasen in Flüssigkeiten.

Bei der an sich bekannten Druckwasserwäsche wird die beladene Waschlösung zuerst entspannt, wobei während der Entspannung aus der Waschlösung entweichendes Methan dem Rohgasstrom zugeführt wird. Anschließend werden aus der Waschlösung mittels Luft CO₂ und H₂S ausgestrippt.

Aus der DE 10 2008 025 971 A1 und DE 10 2008 060 310 A1 sind Verfahren zur Reinigung von Roh- oder Biogas bekannt, die zur Abtrennung von CO₂ Wasser als Waschlösung einsetzen. Zur Reduzierung auftretender Methanverluste wird aus dem verunreinigten Waschwasser in mehreren nachgeschalteten Reinigungsstufen mittels Strippluft oder Strippluft und Sauerstoff im Waschwasser gelöstes Methan abgetrennt, wobei ein sauerstoffhaltiges Strippgas in Brennqualität erhalten wird.

Aus der US 5 842 357 A ist ein Verfahren zur Rückgewinnung von Methan und Kohlendioxid aus Deponiegas bekannt. Das Deponiegas wird in einem Absorber mittels einer im Gegenstrom zugeführten Waschlösung, ein Alkohol (Methanol oder Keton), einer Gaswäscheunterzogen, wobei in der Waschlösung die im Gasstrom enthaltenen Verunreinigungen gelöst werden.

Als Druckwäscheverfahren ist aus der Praxis auch ein sogenanntes Selexol-Verfahren bekannt, bei dem als physkalisch wirkende Lösungsmittel Polyglykolether, insbesondere Glykoldimethylether, zum Einsatz kommen.

Ein Vorteil dieses Verfahrens gegenüber der Druckwasserwäsche ist eine deutlich bessere Löslichkeit von CO₂ in der Waschflüssigkeit (Selexol), wodurch der Verbrauch an Waschflüssigkeit erheblich verringert wird. Ein Nachteil dieser Waschflüssigkeit ist deren deutlich höhere Löslichkeit für organische und anorganische Verbindungen im Vergleich zu Wasser. Weiterhin ist von Nachteil, dass die verunreinigte Waschflüssigkeit nur bei Temperaturen zwischen 55 und 80 °C regeneriert werden kann und vergleichsweise hohe Methanverluste und Verluste anderer brennbarer organischer Stoffe, von denen im Biogas bis zu 0,1% enthalten sind, auftreten.

Bei der Biogasreinigung mit physikalisch wirkenden Waschlösungen wird mit mindestens zwei Kolonnen gearbeitet, die sowohl als Adsorptions- als auch als Desorptionskolonne arbeiten. Die Trocknung der Adsorptionspartikel erfordert einen hohen Wärmeverbrauch. Bei vorgenannten Waschverfahren wird die erreichbare Abscheidung von Kohlendioxid aus Biogas durch die physikalische Löslichkeit von Kohlendioxid und Methan in den Waschmitteln und den Verteilungskoeffizienten (Henry-Koeffizient) bestimmt.

Praktische Untersuchungen haben gezeigt, dass vermutlich während der Gaswäsche in der Waschkolonne sich überlagernde Wasch- und Strippeffekte für Methan auftreten, die sich nachteilig auf den Methanschlupf auswirken.

Versuche, über einen internen CO₂/Methan-Gas-Kreisfauf das CO₂/Methenverhältnis zu verschieben und so die Methananteile im Abgas zu reduzieren, zeigten, dass sich die Methanverluste nur geringfügig reduzieren lassen.

Das anfallende Strippgas aus der Desorptionsstufe muss einer thermischen Nachbehandlung (Verbrennung) zugeführt werden.

Bei den bekannten Verfahren kann der Methanschlupf 3% und mehr betragen.

Der Erfindung liegt die Aufgabe zugrunde, ein Verfahren zur Abtrennung von Kohlendioxid aus Biogas durch Druckwäsche mit einem physikalisch wirkenden Waschmittel zu schaffen, mit dem es möglich ist, den Gehalt an Methan im abgeschiedenen CO₂ deutlich zu reduzieren und die energetische Gesamtbilanz des Verfahrens zu verbessern.

Ferner soll eine zur Durchführung des Verfahrens geeignete, kostengünstige Anlage geschaffen werden.

Erfindungsgemäß wird die Aufgabe durch die im Anspruch 1 angegebenen Merkmale gelöst. Vorteilhafte verfahrenstechnische Weiterbildungen sind Gegenstand der Ansprüche 2 bis 6. Eine zur Durchführung des Verfahrens geeignete Anlage ist Gegenstand des Anspruchs 7. Die Ansprüche 8 und 9 beziehen sich auf weitere Ausgestaltungsvarianten der beanspruchten Anlage

Gemäß der vorgeschlagenen Verfahrensweise wird die nach der Druckwäsche anfallende beladene Waschlösung durch CO₂-Desorption regeneriert und wie folgt weiterbehandelt: Die in der ersten Strippkolonne anfallende beladene Waschlösung wird in einer separaten, zweiten Strippkolonne mittels Strippgas behandelt, wobei aus dieser CO₂ abgetrennt wird. In dieser Stufe fällt vollständig regenerierte Waschlösung an.

Das in der ersten Strippkolonne anfallende Gasgemisch wird einer zweiten Waschkolonne zugeführt und mittels regenerierter Waschlösung in diesem noch enthaltenes CO₂ abgetrennt. Am Kopf dieser Waschkolonne wird Methan mit einem Methangehalt von über 80% abgezogen.

Die am Sumpf der ersten Strippkolonne anfallende Waschlösung wird zusammen mit der aus der zweiten Waschkolonne abgezogenen Waschlösung der zweiten Strippkolonne zugeführt.

Die in der zweiten Waschkolonne anfallende Waschlösung wird der separaten, zweiten Strippkolonne zur Abtrennung von CO₂ zugeführt.

Das während der Entspannungs-/Desorptionsstufe, in der ersten Strippkolonne, anfallende Stripp- bzw. Flashgas wird nicht zur Biogaswaschkolonne zurückgeführt, sondern in einer separaten, zweiten Waschkolonne mit regenerierter Waschlösung gereinigt. In der ersten Desorptionsstufe, der ersten Strippkolonne, wird nur eine kleine Menge an Strippgas eingeleitet, wodurch eine solche Menge an Methan entfernt wird, dass der Methanschlupf deutlich unter 0,2% liegt. Das erhaltene Strippgas wird in einer separaten zweiten Waschkolonne mit regenerierter Waschlösung behandelt, wobei aus diesem CO₂ abgetrennt und Biomethan erhalten wird. Aus den beladenen Waschlösungen der Desorptionskolonne, der ersten Strippkolonne, und gegebenenfalls der zweiten Waschkolonne wird in einer nachgeschalteten separaten, zweiten Strippkolonne mittels Luft CO₂ abgetrennt. Die gereinigten und regenerierten Waschlösungen werden wiederverwendet und können im Kreislaufbetrieb wieder den Waschkolonnen zugeführt werden.

Die vorgeschlagene Verfahrensweise kann insbesondere bei der Druckwasserwäsche und der sogenannten Genosorbwäsche zur Anwendung kommen. Genosorbwäsche ist eine physiklische Wäsche analog der Druckwasserwäsche unter Verwendung von Genosorb (Polyglykoldimethylether) als Waschlösung.

Der entscheidende Nachteil der Gaswäsche-Verfahren auf Basis physikalischer Waschlösungen gegenüber der Arninwäsche (chemische Bindung von CO₂), der sehr hohe Wärmeverbrauch, kann nunmehr deutlich verringert werden.

Bekannte Aminwäschen haben einen Wärmeverbrauch von 0,55 kWh/Nm³ Biogas. Bei einem Methangehalt von 51% Vol.% im Biogas und einem unteren Heizwert von 10 kWh/Nm³ Methan entspricht dies einem Eigenverbrauch von 10,8% des produzierten Methans (entspricht ca. 1,3 Monatsanteile an verbrauchtem Rohstoff pro Jahr). Außerdem ist es erforderlich, die Abwärme zur Wärmerückgewinnung zu verwenden. Bei einem Methanverlust von 0,05% beträgt die Methanverfügbarkeit 89,15%.

Im Vergleich dazu, können mit der vorgeschlagenen Verfahrensweise unter Berücksichtigung der Gewinnung der Prozesswärme aus dem erzeugten Biomethan der Wärmeverbrauch bis auf 0,1 kWh/Nm³ und der Methanverlust bis auf einen Wert von 0,1 % reduziert werden. Dies entspricht einer Methanverfügbarkeit von 97,9%.

Die erfindungsgemäße Verfahrensweise ermöglicht eine fast vollständige Entfernung von Methan aus der Waschlösung. Eine Gasrückführung ist nicht erforderlich. Dadurch verringert sich der Energieverbrauch beim Verdichten des Biogases.

Da im Gesamtprozess kein CO₂ im Kreislauf gefahren wird, verringert sich der Waschmittelbedarf um etwa 10 bis 20%, wodurch sich ein geringerer Energieverbrauch in etwa gleicher Größenordnung ergibt.

In der separaten bzw. zweiten Strippkolonne anfallende regenerierte Waschlösung gelangt wieder in die beiden Waschstufen.

In der ersten Strippkolonne kann außer dem Strippvorgang noch eine Entspannung der beladenen Waschlösung durchgeführt werden, wobei der Systemdruck um 0,5 bis 5 bar abgesenkt wird.

Als physikalisch wirkende Waschlösung können Wasser oder ein Polyglykolether oder ein Gemisch beider Komponenten eingesetzt werden. Waschlösungen dieser Art sind auch unter den Handelsnamen Genosorb oder Selexol bekannt.

Wenn in der ersten Strippkolonne Sauerstoff als Strippgas eingesetzt wird, so kann Sauerstoff zur biologischen Entschwefelung im Fermenter genutzt werden. Hierzu wird das in der zweiten Waschkolonne anfallende Biomethan direkt in den Fermenter zurückgeführt. Als Strippgas, können außer Sauerstoff auch Stickstoff oder Luft eingesetzt werden. Eine zur Durchführung des Verfahrens geeignete Anlage besteht mindestens aus einer ersten Waschkolonne zur Durchführung einer Druckwäsche, einer ersten Strippkolonne, einer zweiten Waschkolonne und einer separaten, zweiten Strippkolonne, also insgesamt vier Kolonnen, die mit für die jeweiligen Verfahrensstufen geeigneten Packungen oder Füllkörpern ausgerüstet sind.

Die erste Waschkolonne ist über eine Leitung mit dem Kopf der ersten Strippkolonne verbunden. Am Kopf der der ersten Strippkolonne ist eine weitere Leitung eingebunden, die mit der zweiten Waschkolonne verbunden ist.

Vom Sumpf der ersten Strippkolonne und der zweiten Waschkolonne führen Leitungen zum Kopf der separaten, zweiten Strippkolonne. Der Sumpf der separaten, zweiten Strippkolonne steht über Leitungen mit der ersten und zweiten Waschkolonne in Verbindung, um regenerierte Waschlösung wieder zurückzuführen.

Die von der zweiten Waschkolonne Biomethan abführende Leitung kann zur Entschwefelung mit dem Fermenter verbunden sein.

In die Biogas zuführende Leitung und in die Leitung, über die Strippgas zur separaten, zweiten Strippkolonne zugeführt wird, ist jeweils ein Verdichter eingebunden.

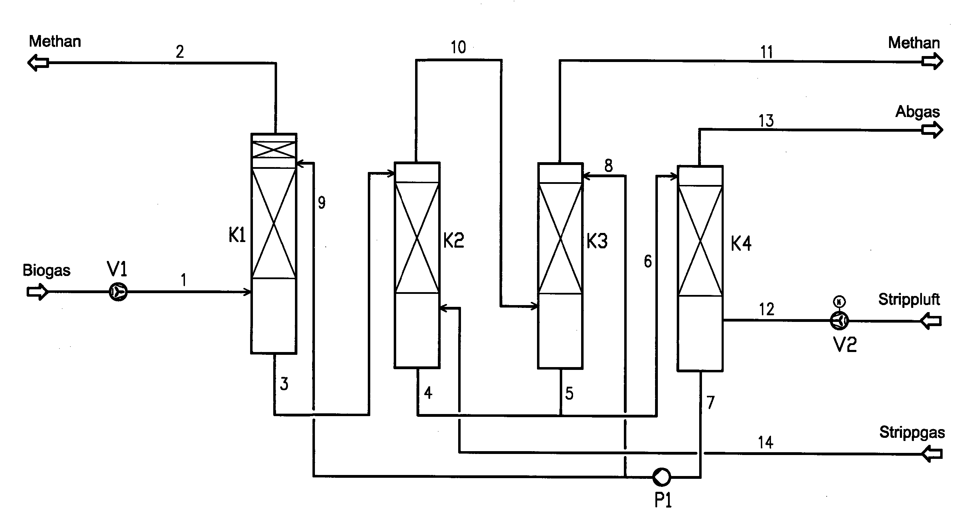

Die Erfindung wird nachstehend unter Bezugnahme auf das in der Zeichnung gezeigte Funktionsschema einer Anlage zur Durchführung des Verfahrens erläutert.

### Beispiel 1

Im Fermenter einer Biogasanlage wird Biogas (Produktionsmenge: 500 Nm³/h) mit folgender Zusammensetzung (Hauptbestandteile) erzeugt:

| | | |
|---|---|---|
| Methan | 51 | Vol.-% |
| Kohlendioxid | 45,3 | Vol.-% |
| Wasser | 3,5 | Vol.-%. |

Erforderlichenfalls wird das Biogas noch entschwefelt, bis auf einen Gehalt von unter 20 ppm.

Danach wird das über die Leitung 1 aus dem Fermenter austretende Biogas mittels eines Verdichters V1 auf einen Druck von 6 bar komprimiert und zur Abtrennung von CO₂ einer Druckwäsche mit der Waschlösung "Genosorb 1753" (Polyethylenglykoldimethylether; Hersteller Clariant GmbH) in einer ersten Waschkolonne K1 unterzogen.

Die Waschkolonne K1 enthält eine Füllkörperschüttung. Anstelle einer Füllkörperschüttung kann auch eine andere geeignete Packung eingesetzt werden.

Das zu reinigende Biogas gelangt über die Leitung 1 mit einer Temperatur von 20 °C in die Waschkolonne K1 und wird mit im Gegenstrom (26,4 m³/h) zugeführter Waschlösung in Kontakt gebracht, wobei im Biogas enthaltenes CO₂ und Wasser in der Waschlösung physikalisch gebunden werden. Die Reinigung erfolgt zuerst mit frischer Waschlösung, die nach erfolgter Beladung und Regenerierung im Kreislauf gefahren wird. Die am Sumpf der Strippkolonne K4 anfallende regenerierte Waschlösung gelangt über die Leitungen 7, 8 und 9, in die eine Pumpe P1 eingebunden ist, in die Waschkolonnen K1 und K3.

Am Kopf der Waschkolonne K1 entweichen über die Leitung 2 244,4 Nm³/h gereinigtes Biogas (Biomethan) mit einer Zusammensetzung von:

| | | |
|---|---|---|
| Methan | 97,9 | Vol.-% |
| Kohlendioxid | 2,1 | Vol.-% |

Die am Kolonnensumpf anfallende Waschlösung mit einer Beladung von 16,8 g/l an Kohlendioxid und 0,43 g/l Methan wird über die Leitung 3 in eine erste Strippkolonne K2 (Desorptionsstufe) geleitet. In diese wird über die Leitung 14 gasförmiger Stickstoff (1 Nm³/h) als Strippgas eingeleitet, der im Gegenstrom mit der Waschlösung in Kontakt gelangt, um in der Waschlösung enthaltenes Methan zu entfernen. Der Arbeitsdruck der Strippkolonne K2 liegt bei ca. 5,6 bar. Unter diesen Bedingungen entweicht am Kopf der Strippkolonne K2 über die Leitung 10 ein Strippgas (40 Nm³/h) mit folgender Zusammensetzung:

| | | |
|---|---|---|
| Methan | 38,93 | Vol.-% |
| Kohlendioxid | 55,98 | Vol.-% |
| Stickstoff | 2,45 | Vol.-% |
| Wasser | Rest. | |

Die am Sumpf der ersten Strippkolonne K2 anfallende Waschlösung hat eine Zusammensetzung von 16,06 g/l an Kohlendioxid und 0,01 g/l Methan und wird über die Leitungen 4 und 6 zusammen mit der aus der zweiten Waschkolonne K3 über die Leitung 5 abgezogenen Waschlösung der zweiten Strippkolonne K4 zugeführt.

In die zweite Strippkolonne K4 wird über die Leitung 12, in die ein zweiter Verdichter V2 eingebunden ist, Strippluft zugeführt, mittels der in der Waschlösung noch enthaltenes CO₂ ausgestrippt wird. Wie bereits erwähnt, hat die am Sumpf dieser Strippkolonne K4 anfallende regenerierte Waschlösung nunmehr wieder die erforderliche Beladungskapazität für einen Einsatz als Waschlösung. Zur Entfernung von CO₂ wird diese nunmehr wieder den Waschkolonnen K1 und K3 zugeführt.

Das anfallende Abgas wird am Kopf der zweiten Strippkolonne K4 über die Leitung 13 abgeführt.

Das aus der ersten Strippkolonne K2 abgezogene Strippgas wird in eine zweite Waschkolonne K3 geleitet, in der mit über die Leitung 8 zugeführter regenerierter Waschlösung (0,7 m³/h) im Strippgas enthaltenes Kohlendioxid entfernt wird.

Das aus der Waschkolonne K3 austretende Gas in einer Menge von 16,5 Nm³/h hat Biomethan-Qualität mit folgender Zusammensetzung:

| | | |
|---|---|---|
| Methan | 92,7 | Vol.-% |
| Kohlendioxid | 1,2 | Vol.-% |
| Stickstoff | 6,1 | Vol.-%. |

Das Biomethan wird über die Leitung 11 abgeführt.

Werden die am Kopf der beiden Waschkolonnen K1 und K3 abgezogenen Mengen an Biomethan gemischt, so entstehen insgesamt 260,9 Nm³/h Biomethan mit einer Zusammensetzung von:

| | | |
|---|---|---|
| Methan | 97,58 | Vol.-% |
| Kohlendioxid | 2,03 | Vol.-% |
| Stickstoff | 0,39 | Vol.-% |

Der Methangehalt im Biomethan kann durch Steigerung der CO₂-Abscheidung in der ersten Waschkolonne K1 noch bis auf ca. 99 Vol.-% erhöht werden.

Im Abgas, das über die Leitung 13 am Kopf der zweiten Strippkolonne abgezogen wird, sind außer Luft noch 221,4 Nm³/h CO₂ und 0,4 Nm³/h Methan enthalten. Dies entspricht einem Methanverlust bzw. Methanschlupf von 0,16 Vol.-%.

### Beispiel 2

Analog wie in Beispiel 1 wird Biogas unter folgenden Bedingungen mit Genosorb gereinigt und die beladene Waschlösung regeneriert.

Das zugeführte Biogas (100 Nm³/h) hat folgende Zusammensetzung:

| | | |
|---|---|---|
| Methan | 58 | Vol.-% |
| Kohlendioxid | 37,9 | Vol.-% |
| Wasser | 4,1 | Vol.-%. |

Das Biogas wird auf einen Druck von 5 bar komprimiert und der Waschkolonne K1 mit einer Temperatur von 20 °C zugeführt. Der Waschkolonne K1 werden im Gegenstrom 6,2 m³/h an Waschlösung zugeführt. Am Kopf der Waschkolonne K1 wird Biomethan (56 Nm³/h) mit folgender Zusammensetzung abgezogen:

| | | |
|---|---|---|
| Methan | 98,3 | Vol.-%. |
| Kohlendioxid | 1,7 | Vol.-%. |

Die austretende Waschlösung hat eine Beladung von 11,8 g/l an Kohlendioxid und 0,41 g/l Methan und wird in der Strippkolonne K2 mit 0,5 Nm³/h gasförmigem Stickstoff behandelt. Der Arbeitsdruck der Strippkolonne liegt bei 4,0 bar. Unter diesen Bedingungen entweicht am Kopf der Kolonne K2 Strippgas (11 Nm³/h) mit folgender Zusammensetzung:

| | | |
|---|---|---|
| Methan | 31,7 | Vol.-% |
| Kohlendioxid | 62,8 | Vol.-% |
| Stickstoff | 4,5 | Vol.-% |
| Wasser | Rest. | |

Die austretende Waschlösung mit einer Zusammensetzung von 9,6 g/l an Kohlendioxid und 0,005 g/l Methan wird der zweiten Strippkolonne K4 zugeführt.

Aus dem Strippgas der ersten Strippkolonne K2 wird in der Waschkolonne K3 mit regenerierter Waschlösung (0,9 m³/h) Kohlendioxid entfernt.

Das aus der Waschkolonne K3 austretende Biomethan (4,1 Nm³/h) hat folgende Zusammensetzung:

| | | |
|---|---|---|
| Methan | 82,9 | Vol.-% |
| Kohlendioxid | 4,8 | Vol.-% |
| Stickstoff | 12,2 | Vol.-%. |

Die am Kopf der Waschkolonne K1 und K3 anfallenden Biomethanmengen werden zusammengeführt. Es entstehen insgesamt 60,1 Nm³/h Biomethan mit folgender Zusammensetzung:

| | | |
|---|---|---|
| Methan | 97,17 | Vol.-% |
| Kohlendioxid | 2,00 | Vol.-% |
| Stickstoff | 0,83 | Vol.-%. |

Die Waschlösungen aus der Strippkolonne K2 und der Waschkolonne K3 werden zusammen der Strippkolonne K4 zugeführt. In dieser wird mittels Strippluft in der Waschlösung enthaltenes Kohlendioxid entfernt. Die regenerierte Waschlösung kann nunmehr wieder in die Waschkolonnen K1 und K3 zurückgeführt werden.

Im abgeführten Stripp- bzw. Abgas der Strippkolonne K4 sind außer Luft noch 36,7 Nm³/h CO₂ und 0,06 Nm³/h Methan enthalten. Dies entspricht einem Methanverlust von 0,103%.

### Beispiel 3

Dieses Beispiel unterscheidet sich von den vorhergehenden Beispielen dadurch, dass am Kopf der zweiten Waschkolonne K3 abströmendes Biomethan (4,1 Nm³/h) zusammen mit 0,5 Nm³/h Sauerstoff in den Fermenter eingetragen wird. Der Sauerstoff wird zur biologischen Entschwefelung im Fermenter benötigt und reduziert sich auf einen Wert von 0,2 Nm³/h.

Das aus dem Fermenter abgezogene Biogas (100 Nm³/h) hat die gleiche Zusammensetzung wie das Biogas gemäß Beispiel 2 und wird auch unter gleichen Bedingungen (wie in Beispiel 2) einer Gaswäsche unterzogen.

Am Kopf der Waschkolonne K1 wird Biomethan (59,4 Nm³/h) mit folgender Zusammensetzung abgezogen:

| | | |
|---|---|---|
| Methan | 98,2 | Vol.-% |
| Kohlendioxid | 1,5 | Vol.% |
| Sauerstoff | 0,3 | Vol.-% |

Die austretende Waschlösung hat eine Beladung von 11,8 g/l an Kohlendioxid und 0,41 g/l Methan und wird der Strippkolonne K2 zugeführt. In diese werden im Gegenstrom zur Waschlösung 0,5 Nm³/h gasförmiger Sauerstoff eingeleitet. Der Arbeitsdruck der Strippkolonne K2 liegt bei 4,0 bar. Unter diesen Bedingungen entweicht am Kopf der Strippkolonne K2 ein Strippgas (11 Nm³/h) mit folgender Zusammensetzung:

| | | |
|---|---|---|
| Methan | 31,7 | Vol.-% |
| Kohlendioxid | 62,8 | Vol.-% |
| Sauerstoff | 4,5 | Vol.-% |
| Wasser | Rest. | |

Die austretende Waschlösung mit einer Zusammensetzung von 9,6 g/l an Kohlendioxid und 0,005 g/l Methan wird der zweiten Strippkolonne K4 zugeführt.

Aus dem Strippgas der ersten Strippkolonne K2 wird in der Waschkolonne K3 mit regenerierter Waschlösung (0,9 m³/h) Kohlendioxid entfernt.

Das aus der Waschkolonne K3 austretende Biomethan (4,1 Nm³/h) hat folgende Zusammensetzung:

| | | |
|---|---|---|
| Methan | 82,9 | Vol.-% |
| Kohlendioxid | 4,8 | Vol.-% |
| Sauerstoff | 12,2 | Vol.-%. |

Wie bereits eingangs erwähnt, wird dieses Biomethan zum Fermenter zurückgeführt und dort vermischt. Der Sauerstoffanteil wird zur biologischen Entschwefelung genutzt.

Die weitere Behandlung der Waschlösungen aus den Kolonnen K2 und K3 erfolgt analog wie in Beispiel 2.

## Patentansprüche

1. Verfahren zur Abtrennung von Kohlendioxid aus Biogas, wobei aus dem Biogas durch Druckwäsche in einer ersten Waschkolonne (K1) mittels einer im Gegenstrom zugeführten physikalisch wirkenden Waschlösung Kohlendioxid abgetrennt und in der Waschlösung gelöst wird, gereinigtes Biogas abgezogen und die beladene Waschlösung durch CO₂-Abtrennung in einer ersten Strippkolonne (K2) regeneriert wird, wobei ein methan- und kohlendioxidhaltiges Gasgemisch und eine mit CO₂ restbeladene Waschlösung anfallen, **dadurch gekennzeichnet, dass**
a) die in der ersten Strippkolonne (K2) anfallende restbeladene Waschlösung in einer separaten, zweiten Strippkolonne (K4) mittels Strippgas behandelt wird, wobei aus dieser CO₂ abgetrennt wird und in dieser Stufe vollständig regenerierte Waschlösung anfällt,
b) das in der ersten Strippkolonne (K2) anfallende Gasgemisch einer zweiten Waschkolonne (K3) zugeführt und mittels regenerierter Waschlösung in diesem noch enthaltenes CO₂ abgetrennt wird, wobei am Kopf der zweiten Waschkolonne (K3) Methan abgezogen wird, und
c) die am Sumpf der ersten Strippkolonne (K2) anfallende Waschlösung zusammen mit der aus der zweiten Waschkolonne (K3) abgezogenen Waschlösung der zweiten Strippkolonne (K4) zugeführt wird.

2. Verfahren nach Anspruch 1, **dadurch gekennzeichnet, dass** in der separaten, zweiten Strippkolonne (K4) anfallende regenerierte Waschlösung wieder den beiden Waschkolonnen (K1, K3) zugeführt wird.

3. Verfahren nach einem der Ansprüche 1 oder 2, **dadurch gekennzeichnet, dass** in der ersten Strippkolonne (K2) noch eine Entspannung der beladenen Waschlösung durchgeführt wird, wobei der Systemdruck um einen Wert von 0,5 bis 5 bar abgesenkt wird.

4. Verfahren nach einem der Ansprüche 1 bis 3, **dadurch gekennzeichnet, dass** als Waschlösung Wasser oder ein Polyglykolether oder ein Gemisch beider Komponenten eingesetzt wird.

5. Verfahren nach einem der Ansprüche 1 bis 4, **dadurch gekennzeichnet, dass** in der ersten Strippkolonne (K2) als Strippgas Sauerstoff eingesetzt wird und das in der zweiten Waschkolonne (K3) anfallende Biomethan in einen Fermenter zurückgeführt wird, wobei der im Biomethan enthaltene Sauerstoff zur biologischen Entschwefelung des im Fermenter erzeugten Biogases genutzt wird.

6. Verfahren nach einem der Ansprüche 1 bis 5, **dadurch gekennzeichnet, dass** in den Strippkolonnen (K2, K4) als Strippgas Stickstoff, Sauerstoff oder Luft eingesetzt werden.

7. Anlage zur Durchführung des Verfahrens nach einem der Ansprüche 1 bis 6, **dadurch gekennzeichnet, dass** diese mindestens aus einer ersten Waschkolonne (K1) zur Durchführung einer Druckwäsche, einer ersten Strippkolonne (K2), einer zweiten Waschkolonne (K3) und einer separaten, zweiten Strippkolonne (K4) besteht, wobei die erste Waschkolonne (K1) über eine Leitung (3) mit dem Kopf der ersten Strippkolonne (K2) verbunden ist, am Kopf der ersten Strippkolonne (K2) eine Leitung (10) eingebunden ist, die mit der zweiten Waschkolonne (K3) verbunden ist, der Sumpf der ersten Strippkolonne (K2) und der zweiten Waschkolonne (K3) über Leitungen (4, 5, 6) mit dem Kopf der separaten, zweiten Strippkolonne (K4) verbunden sind und vom Sumpf der separaten Strippkolonne (K4) eine regenerierte Waschlösung führende Leitung (7) abzweigt, die über Leitungen (8, 9) mit der ersten und zweiten Waschkolonne (K1, K3) in Verbindung steht.

8. Anlage nach Anspruch 7, ferner aufweisend einen Fermenter, **dadurch gekennzeichnet, dass** die von der zweiten Waschkolonne (K3) Biomethan abführende Leitung (11) mit dem Fermenter verbunden ist.

9. Anlage nach einem der Ansprüche 7 oder 8, **dadurch gekennzeichnet, dass** in die Biogas zuführende Leitung (1) und in die Leitung (12), über die Strippgas zur separaten Strippkolonne (K4) zugeführt wird, jeweils ein Verdichter (V1, V2) eingebunden ist.

## Claims

1. A method for separating carbon dioxide from biogas, wherein carbon dioxide is separated from the biogas by means of pressure scrubbing in a first scrubbing column (K1) using a physically active scrubbing solution fed in counter-current, and is dissolved in the scrubbing solution, the scrubbed biogas is withdrawn and the charged scrubbing solution is regenerated in a first stripping column (K2) by means of CO₂ separation, wherein a gas mixture containing methane and carbon dioxide and a scrubbing solution residually charged with CO₂ are obtained, **characterized in that**
a) the residually charged scrubbing solution obtained in the first stripping column (K2) is treated in a separate, second stripping column (K4) using stripping gas, wherein CO₂ is separated therefrom and that a fully regenerated scrubbing solution is obtained in this step,
b) the gas mixture obtained in the first stripping column (K2) is fed to a second scrubbing column (K3) and CO₂ still contained therein is separated using the regenerated scrubbing solution, wherein methane is withdrawn at the head of the second scrubbing column (K3), and
c) the scrubbing solution obtained at the sump of the first stripping column (K2) is fed together with the scrubbing solution withdrawn from the second scrubbing column (K3) to the second stripping column (K4).

2. The method according to claim 1, **characterized in that** the regenerated solution obtained in the separate second stripping column (K4) is fed back to the two scrubbing columns (K1, K3).

3. The method according to one of the claims 1 or 2, **characterized in that** the charged scrubbing solution is furthermore expanded in the first stripping column (K2), wherein the system pressure is reduced by a value of 0.5 to 5 bar.

4. The method according to one of the claims 1 to 3, **characterized in that** water or polyglycol ether or a mixture of both components is used as a scrubbing solution.

5. The method according to one of the claims 1 to 4, **characterized in that** oxygen is used as a stripping gas in the first stripping column (K2) and the biomethane obtained in the second scrubbing column (K3) is fed back into a fermenter, wherein the oxygen contained in the biomethane is used for biological desulfurization of the biogas produced in the fermenter.

6. The method according to one of the claims 1 to 5, **characterized in that** nitrogen, oxygen or air is used as a stripping gas in the stripping columns (K2, K4).

7. A plant for carrying out the method according to one of the claims 1 to 6, **characterized in that** it consists at least of one first scrubbing column (K1) for carrying out pressure scrubbing, a first stripping column (K2), a second scrubbing column (K3) and a separate, second stripping column (K4), wherein the first scrubbing column (K1) is connected to the head of the first stripping column (K2) by way of a pipe (3), a pipe (10) is integrated in the head of the first stripping column (K2) and is connected with the second scrubbing column (K3), the sump of the first stripping column (K2) and of the second scrubbing column (K3) are connected to the head of the separate, second stripping column (K4) via pipes (4, 5, 6) and a pipe (7) carrying a regenerated scrubbing solution branches off from the sump of the separate stripping column (K4) and connects with the first and second scrubbing column (K1, K3) via pipes (8, 9)

8. The plant according to claim 7, further comprising a fermenter, **characterized in that** the pipe (11) that discharges biomethane from the second scrubbing column (K3) is connected to the fermenter.

9. The plant according to one of the claims 7 or 8, **characterized in that** respectively one compressor (V1, V2) is integrated in the pipe (1) supplying the biogas and in the pipe (12), by way of which the stripping gas is fed into the separate stripping column (K4).

## Revendications

1. Procédé de séparation de dioxyde de carbone de biogaz, où le dioxyde de carbone est séparé du biogaz par lavage sous pression dans une première colonne de lavage (K1) à l'aide d'une solution de lavage acheminée à contre-courant et agissant physiquement et est dissout dans la solution de lavage, le biogaz nettoyé est prélevé et la solution de lavage chargée est régénérée dans une première colonne d'épuration (K2) par séparation du CO₂, où un mélange gazeux contenant du méthane et du dioxyde de carbone et une solution de lavage résiduellement chargée de CO₂ sont produites, **caractérisé en ce que**
a) la solution de lavage résiduellement chargée produite dans la première colonne d'épuration (K2) est traitée dans une seconde colonne d'épuration (K4) distincte à l'aide de gaz d'épuration, où du CO₂ y est séparé et une solution de lavage totalement régénérée est produite lors de cette étape,
b) le mélange de gaz produit dans la première colonne d'épuration (K2) est acheminé vers une seconde colonne de lavage (K3) et du CO₂ encore présent dans celui-ci est séparé à l'aide d'une solution de lavage régénérée, où du méthane est prélevé au niveau de la tête de la seconde colonne de lavage (K3), et
c) la solution de lavage produite dans le fond de la première colonne d'épuration (K2) ainsi que la solution de lavage prélevée dans la seconde colonne de lavage (K3) sont acheminées vers la seconde colonne d'épuration (K4).

2. Procédé selon la revendication 1, **caractérisé en ce que** la solution de lavage régénérée obtenue dans la seconde colonne de lavage (K4) distincte est réacheminée vers les deux colonnes de lavage (K1, K3).

3. Procédé selon l'une quelconque des revendications 1 ou 2, **caractérisé en ce qu'**une détente de la solution de lavage chargée est encore effectuée dans la première colonne d'épuration (K2), où la pression du système est réduite d'une valeur de 0,5 à 5 bar.

4. Procédé selon l'une des revendications 1 à 3, **caractérisé en ce que** de l'eau ou un éther de polyglycol ou un mélange de ces deux composants est utilisé en tant que solution de lavage.

5. Procédé selon l'une des revendications 1 à 4, **caractérisé en ce que** de l'oxygène est utilisé en tant que gaz d'épuration dans la première colonne d'épuration (K2) et le biométhane produit dans la second colonne de lavage (K3) est réacheminé dans un fermenteur, où l'oxygène contenu dans le biométhane est utilisé pour la désulfuration biologique du biogaz produit dans le fermenteur.

6. Procédé selon l'une des revendications 1 à 5, **caractérisé en ce que** de l'azote, de l'oxygène ou de l'air est utilisé dans les colonnes d'épuration (K2, K4) en tant que gaz d'épuration.

7. Installation pour la mise en oeuvre du procédé selon l'une des revendications 1 à 6, **caractérisé en ce qu'**elle se compose d'au moins une première colonne de lavage (K1) pour la mise en oeuvre d'un lavage sous pression, d'une première colonne d'épuration (K2), d'une seconde colonne de lavage (K3) et d'une seconde colonne d'épuration (K4) distincte, où la première colonne de lavage (K1) est reliée avec la tête de la première colonne d'épuration (K2) via un tuyau (3), un tuyau (10) est intégré dans la tête de la première colonne d'épuration (K2) et relié avec la seconde colonne de lavage (K3), le fond de la première colonne d'épuration (K2) et de la seconde colonne de lavage (K3) sont reliés via des tuyaux (4, 5, 6) avec la tête de la seconde colonne d'épuration distincte (K4) et un tuyau (7) conduisant une solution de lavage épurée bifurque du fond de la colonne d'épuration séparée (K4) et est reliée avec la première et seconde colonne de lavage (K1, K3) via des tuyaux (8, 9).

8. Installation selon la revendication 7, comportant en outre un fermenteur, **caractérisée en ce que** le tuyau (11) évacuant le biométhane de la seconde colonne de lavage (K3) est reliée au fermenteur.

9. Installation selon l'une des revendications 7 ou 8, **caractérisée en ce qu'**un compresseur (V1, V2) est respectivement intégré dans le tuyau (1) acheminant le biogaz et dans le tuyau (12) par lequel le gaz d'épuration est acheminé vers la colonne d'épuration (K4) distincte.
